# EUROPEAN PATENT APPLICATION

(11) **EP 3 721 799 A1**
(43) Date of publication of application: **14.10.2020**
(21) Application number: 20174673.2
(22) Date of filing: 15.12.2014
(51) Int. Cl.: A61B 5/055, A61K 38/06, A61K 49/06, A61P 25/00, A61P 25/24, A61P 25/28

(54) **METHODS OF TREATING BRAIN DISORDERS OR IDENTIFYING BIOMARKERS RELATED THERETO**

(30) Priority: 13.12.2013 US 201361915835 P
(62) Divisional of application: 14870211.1
(71) Applicant: Northwestern University, Evanston, IL 60208 (US)
(72) Inventor: Moskal, Joseph, Evanston, IL Illinois 60201 (US)
(74) Representative: Hoffmann Eitle

(57) **Abstract**

The present disclosure relates in part to methods of treating cognitive disorders and/or identifying biomarkers.

## Description

### CROSS REFERENCE TO RELATED APPLICATIONS

This application claims the benefit of United States Provisional Application No. 61/915,835, filed on December 13, 2013, which is incorporated herein by reference in its entirety.

### BACKGROUND

The N-methyl-D-aspartate (NMDA) receptor (NMDAR) has been implicated in neurodegenerative disorders including stroke-related brain cell death, convulsive disorders, and learning and memory. NMDAR also plays a central role in modulating normal synaptic transmission, synaptic plasticity, and excitotoxicity in the central nervous system. The NMDAR is further involved in Long-term potentiation (LTP).

The NMDAR is activated by the binding of NMDA, glutamate (Glu), and aspartate (Asp). It is competitively antagonized by D-2-amino-5-phosphonovalerate (D-AP5; D-APV), and non-competitively antagonized by phenylcyclidine (PCP), and MK-801. Most interestingly, the NMDAR is co-activated by glycine (Gly) (Kozikowski et al., 1990, Journal of Medicinal Chemistry 33:1561-1571). The binding of glycine occurs at an allosteric regulatory site on the NMDAR complex, and this increases both the duration of channel open time, and the frequency of the opening of the NMDAR channel.

NMDA-modulating small molecule agonist and antagonist compounds have been developed for potential therapeutic use. For example, recent human clinical studies have identified NMDAR as a novel target of high interest for treatment of depression. These studies conducted using known NMDAR antagonists CPC-101,606 and ketamine have shown significant reductions in the Hamilton Depression Rating Score in patients suffering with refractory depression. Although the efficacy was significant, the side effects of using these NDMAR antagonists were severe. Such compounds may also have utility for learning or for treatment of cognitive disorders.

However, there remains a need for understanding how these compounds work and/or identification of patient populations in need of such treatment.

Recently, an improved partial agonist of NMDAR, termed as GLYX-13, has been reported. GLYX-13 exhibits nootropic, neuroprotective and antinociceptive activity, and enhances learning, memory and cognition *in vivo.* GLYX-13, has also been shown to exhibit rapid-acting, robust, and sustained antidepressant activity and to lack the pyschotomimetic side effects associated with other drugs and mechanisms that target the NMDA receptor.

### SUMMARY

In one aspect, the present disclosure relates in part to methods of treating a cognitive disorder or enhancing cognitive function and/or learning in a patient in need thereof, comprising: generating a MRI signal that is a measurement of brain activity in a patient; identifying the signal as a normal or abnormal brain state of the patient; and administering to the patient an effective amount of a NMDAR partial agonist, e.g., GLYX-13, based on the signal identification. Some embodiments can include one or more of the following features, which can further be combined with one or more other features disclosed herein. Generating an MRI signal can include using functional magnetic resonance imaging, e.g., the functional magnetic resonance imaging can include blood-oxygen-level dependent contrast imaging. The brain activity can be neural activation, e.g., neural activation in learning and/or memory related regions of the brain.

In another aspect, a method is provided for identifying a biomarker related to neural activation, learning, or memory or identifying a patient population who is more susceptible to such disorders, comprising administering a NMDAR partial agonist, e.g., GLYX-13 to a subject (e.g., an animal, e.g., human or rodent); imaging the animal using functional magnetic resonance to create measurable activity such as blood oxygen levels; analyzing the activity; and identifying the biomarker as result of the activity. Some embodiments can include one or more of the following features, which can further be combined with one or more other features disclosed herein. Using functional magnetic resonance to create measurable activity can include using blood-oxygen-level dependent contrast imaging. The measurable activity can include changes in blood flow and/or blood oxidation in one or more regions of the brain in the animal. The biomarker can be a blood-oxygen-level dependent contrast signal in one or more regions of the brain in the animal. The method can further include associating a patient or subpopulation of patients with the biomarker. The method can further include one or both of the following: (a) determining whether GLYX-13 would be therapeutically effective for treating a cognitive or mental disorder; and (b) determining susceptibility/receptivity in patient or a subpopulation of patients suffering from a cognitive or mental disorder (e.g., depression, e.g., refractory depression).

In a further aspect, a method is provided for treating a disorder selected from the group consisting of epilepsy, AIDS dementia, multiple system atrophy, progressive supra-nuclear palsy, Friedrich's ataxia, autism, fragile X syndrome, tuberous sclerosis, attention deficit disorder, olivio-ponto-ccrcbcllar atrophy, cerebral palsy, drug-induced optic neuritis, peripheral neuropathy, myelopathy, ischemic retinopathy, glaucoma, cardiac arrest, behavior disorders, impulse control disorders, attention deficit disorder, attention deficit hyperactivity disorder, schizophrenia, anxiety, amelioration of opiate, nicotine and/or ethanol addiction, spinal cord injury, diabetic retinopathy, traumatic brain injury, post-traumatic stress syndrome, Huntington's chorea, Alzheimer's disease, memory loss that accompanies early stage Alzheimer's disease, depression conditions, Major Depressive Disorder, Dysthymic Disorder, Psychotic depression, Postpartum depression, Seasonal affective disorder (SAD), mood disorder, depressions caused by chronic medical conditions such as cancer or chronic pain, chemotherapy, chronic stress, Bipolar disorder, and manic depressive disorder, in a patient in need thereof, comprising: generating a MRI signal that is a measurement of brain activity in a patient; identifying the signal as a normal or abnormal brain state of the patient; and administering to the patient an effective amount of a NMDAR partial agonist, e.g., GLYX-13, based on the signal identification. Some embodiments can include one or more of the following features, which can further be combined with one or more other features disclosed herein. Generating an MRI signal can include using functional magnetic resonance imaging, e.g., the functional magnetic resonance imaging can include blood-oxygen-level dependent contrast imaging.

In still another aspect, a method is provided for identifying a biomarker related to a disorder selected from the group consisting of epilepsy, AIDS dementia, multiple system atrophy, progressive supra-nuclear palsy, Friedrich's ataxia, autism, fragile X syndrome, tuberous sclerosis, attention deficit disorder, olivio-ponto-ccrcbcllar atrophy, cerebral palsy, drug-induccd optic neuritis, peripheral neuropathy, myelopathy, ischemic retinopathy, glaucoma, cardiac arrest, behavior disorders, impulse control disorders, attention deficit disorder, attention deficit hyperactivity disorder, schizophrenia, anxiety, amelioration of opiate, nicotine and/or ethanol addiction, spinal cord injury, diabetic retinopathy, traumatic brain injury, post-traumatic stress syndrome, Huntington's chorea, Alzheimer's disease, memory loss that accompanies early stage Alzheimer's disease, depression conditions, Major Depressive Disorder, Dysthymic Disorder, Psychotic depression, Postpartum depression, Seasonal affective disorder (SAD), mood disorder, depressions caused by chronic medical conditions such as cancer or chronic pain, chemotherapy, chronic stress, Bipolar disorder, and manic depressive disorder, or identifying a patient population who is more susceptible to such disorders, comprising administering a NMDAR partial agonist, e.g., GLYX-13 to a subject (e.g., an animal, e.g., human or rodent); imaging the animal using functional magnetic resonance to create measurable activity such as blood oxygen levels; analyzing the activity; and identifying the biomarker as result of the activity. Some embodiments can include one or more of the following features, which can further be combined with one or more other features disclosed herein. Using functional magnetic resonance to create measurable activity can include using blood-oxygen-level dependent contrast imaging. The measurable activity can include changes in blood flow and/or blood oxidation in one or more regions of the brain in the animal. The biomarker can be a blood-oxygen-level dependent contrast signal in one or more regions of the brain in the animal. The method can further include associating a patient or subpopulation of patients with the biomarker. The method can further include one or both of the following: (a) determining whether GLYX-13 would be therapeutically effective for treating the disorder; and (b) determining susceptibility/receptivity in patient or a subpopulation of patients suffering from the disorder.

In still another aspect, a method is provided for tracking treatment progress and/or treatment endpoints in a patient suffering from a cognitive or mental disorder comprising generating a MRI signal that is a measurement of brain activity in a patient; identifying the signal as a normal or abnormal brain state of the patient; and administering to the patient an effective amount of GLYX-13 based on the signal identification. Some embodiments can include one or more of the following features, which can further be combined with one or more other features disclosed herein. Generating an MRI signal can include using functional magnetic resonance imaging, e.g., the functional magnetic resonance imaging can include blood-oxygen-level dependent contrast imaging.

### BRIEF DESCRIPTION OF THE DRAWINGS

FIG. 1 is a flow diagram summarizing the study design for the study described in Example 2.
FIG. 2 is a flow diagram summarizing the study flow for the study described in Example 2.
FIG. 3 is a diagram that summarizes the design of the item category association task used in the study described in Example 2.
FIG. 4 is a diagram that summarizes the second level fixed effects analyses performed in the study described in Example 2.
FIG. 5 is a graph showing that GLYX-13 and Placebo treated subjects show a robust and comparable learning effect across cycle.
FIG. 6 provides a series of fMRI images of regions showing significant change in BOLD activation across learning cycles.
FIGS. 7A-7F provide a series of fMRI images of regions showing that among identified regions, six demonstrated significant group by cycle interaction effects on extracted mean % signal change, all of which demonstrated enhanced activation among GLYX-13 vs. Placebo treated individuals.

### DETAILED DESCRIPTION

The present disclosure relates in part to methods for identifying a biomarker related to neural activation, learning, or memory or identifying a patient population who is more susceptible to such disorders, comprising administering GLYX-13 to an animal; imaging the animal using functional magnetic resonance to create measurable activity such as blood oxygen levels; analyzing the activity, and identifying the biomarker as result of the activity.

Also provided herein arc methods of treating a cognitive disorder or enhancing cognitive function and/or learning in a patient in need thereof, comprising: generating a MRI signal that is a measurement of brain activity in a patient; identifying the signal as a normal or abnormal brain state of the patient; and administering to the patient an effective amount of GLYX-13 based on the signal identification.

Functional magnetic resonance imaging or functional MRI (fMRI) is a functional neuroimaging procedure using MRI technology that measures brain activity by detecting associated changes in blood flow. This technique relies on the fact that cerebral blood flow and neuronal activation are coupled. When an area of the brain is in use, blood flow to that region also increases. The primary form of fMRI uses the Blood-oxygen-level dependent (BOLD) contrast. fMRI can in some embodiments, also be combined and complemented with other measures of brain physiology such as EEG and NIRS. Other methods may largely use biomarkers other than the BOLD signal.

Contemplated methods include a methods of treating, or method of identifying biomarkers of, autism and/or an autism spectrum disorder. In some embodiments, patients suffering from autism also suffer from another medical condition, such as Fragile X syndrome, tuberous sclerosis, congenital rubella syndrome, and untreated phenylketonuria.

In another embodiment, methods of treating, or method of identifying biomarkers of, wherein the disorder is selected from group consisting of: epilepsy, AIDS dementia, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, and impulse control disorders that includes administering an identified compound.

In an embodiment, contemplated herein are methods of treating or identifying biomarkers related to attention deficit disorder, ADHD (attention deficit hyperactivity disorder), schizophrenia, anxiety, amelioration of opiate, nicotine and/or ethanol addiction (e.g., method of treating such addiction or ameliorating the side effects of withdrawing from such addiction), spinal cord injury diabetic retinopathy, traumatic brain injury, post-traumatic stress syndrome and/or Huntington's chorea, in a patient in need thereof or identifying a biomarker for one or more of these disorders, that includes administering an identified compound. For example, patients suffering from schizophrenia, addiction (e.g. ethanol or opiate), autism, Huntington's chorea, traumatic brain injury, spinal cord injury, post-traumatic stress syndrome and diabetic retinopathy may all be suffering from altered NMDA receptor expression or functions.

In another embodiment, disclosed methods relate to Alzheimer's disease, or e.g., treatment of memory loss that e.g., accompanies early stage Alzheimer's disease. In another embodiment, disclosed methods may relate to common depression conditions including Major Depressive Disorder and Dysthymic Disorder. Other depression conditions develop under unique circumstances. Such depression conditions include but are not limited to Psychotic depression, Postpartum depression, Seasonal affective disorder (SAD), mood disorder, depressions caused by chronic medical conditions such as cancer or chronic pain, chemotherapy, chronic stress, post traumatic stress disorders, and Bipolar disorder (or manic depressive disorder). Refractory depression occurs in patients suffering from depression who are resistant to standard pharmacological treatments, including tricyclic antidepressants, MAOIs, SSRIs, and double and triple uptake inhibitors and/or anxiolytic drugs, as well non-pharmacological treatments such as psychotherapy, electroconvulsive therapy, vagus nerve stimulation and/or transcranial magnetic stimulation. Treatment resistant-patient or animals (e.g. humans) are contemplated for treatment or identified as one who fails to experience alleviation of one or more symptoms of depression (e.g., persistent anxious or sad feelings, feelings of helplessness, hopelessness, pessimism) despite undergoing one or more standard pharmacological or non-pharmacological treatment. In certain embodiments, a treatment-resistant patient is one who fails to experience alleviation of one or more symptoms of depression despite undergoing treatment with two different antidepressant drugs. In other embodiments, a treatment-resistant patient is one who fails to experience alleviation of one or more symptoms of depression despite undergoing treatment with four different antidepressant drugs. A treatment-resistant patient may also be identified as one who is unwilling or unable to tolerate the side effects of one or more standard pharmacological or non-pharmacological treatment. In certain embodiments, methods for treating refractory depression by administering an effective amount of an identified compound to a treatment-resistant patient in need thereof are contemplated. In an embodiment, methods of treating depression is contemplated when a patient has suffered depression for e.g. 5, 6, 7, 8 or more weeks, or for a month or more..

In another embodiment, methods of treating a disorder in a patient need thereof are contemplated, wherein the disorder is selected from group consisting of: epilepsy, AIDS dementia, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, and impulse control disorders that includes administering an identified compound.

Treating" includes any effect, e.g., lessening, reducing, modulating, or eliminating, that results in the improvement of the condition, disease, disorder and the like. "Individual," "patient," or "subject" are used interchangeably and include any animal, including mammals, preferably mice, rats, other rodents, rabbits, dogs, cats, swine, cattle, sheep, horses, or primates, and most preferably humans.

The term "effective amount" refers to an amount of the subject component, e.g., GLYX-13 (or a composition containing GLYX-13) that will elicit the biological or medical response of a tissue, system, animal or human that is being sought by the researcher, veterinarian, medical doctor or other clinician.

As used herein, the term "GLYX peptide" refers to a peptide having NMDAR glycine-site partial agonist/antagonist activity. GLYX peptides may be obtained by well-known recombinant or synthetic methods such as those described in US Patents 5,763,393 and 4,086,196 herein incorporated by reference. In some embodiments, GLYX refers to a tetrapeptide having the amino acid sequence Thr-Pro-Pro-Thr (SEQ ID NO: 13), or L-threonyl-L-prolyl-L-prolyl-L-threonine amide. In some embodiments, candidate compounds have the same microarray results as GLYX-13 and/or the below compounds.

For example, GLYX-13 refers to the compound depicted as:

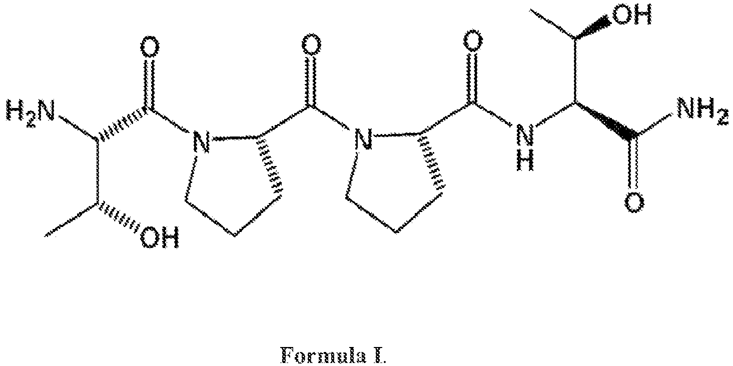

Also contemplated are polymorphs, homologs, hydrates, solvates, free bases, and/or suitable salt forms of GLYX 13 such as, but not limited to, the acetate salt. The peptide may be cyclyzed or non-cyclyzed form as further described in US 5,763,393. In some embodiments, an a GLYX-13 analog may include an insertion or deletion of a moiety on one or more of the Thr or Pro groups such as a deletion of CH₂, OH, or NH₂ moiety. In other embodiments, GLYX-13 may be optionally substituted with one or more halogens, C₁-C₃ alkyl (optionally substituted with halogen or amino), hydroxyl, and/or amino. Glycine-site partial agonist of the NMDAR are disclosed in US 5,763,393, US 6,107,271, and Wood et al., NeuroReport, 19, 1059-1061, 2008, the entire contents of which are herein incorporated by reference.

In some embodiments, a therapeutically effective amount of GLYX-13, e.g., for adult human treatment, can be in the range of from about 0.01 mg/kg to about 1000 mg/kg per administration (e.g., about 0.01 mg/kg to about 100 mg/kg, about 0.01 mg/kg to about 50 mg/kg, about 0.01 mg/kg to about 25 mg/kg, about 0.01 mg/kg to about 10 mg/kg, about 0.1 mg/kg to about 100 mg/kg, about 0.1 mg/kg to about 50 mg/kg, about 0.1 mg/kg to about 50 mg/kg, about 0.1 mg/kg to about 10 mg/kg, about 1 mg/kg to about 100 mg/kg, about 1 mg/kg to about 50 mg/kg, about 1 mg/kg to about 50 mg/kg per day, about 1 mg/kg to about 10 mg/kg, or about 1 mg/kg to about 10 mg/kg per administration, e.g., once a week, twice a week or three times a week and/or as described anywhere herein). The dosage of GLYX-13 may be at any dosage including, but not limited to, about 1 ug/kg, 25 ug/kg, 50 ug/kg, 75 ug/kg, 100 u ug/kg, 125 ug/kg, 150 ug/kg, 175 ug/kg, 200 ug/kg, 225 ug/kg, 250 ug/kg, 275 ug/kg, 300 ug/kg, 325 ug/kg, 350 ug/kg, 375 ug/kg, 400 ug/kg, 425 ug/kg, 450 ug/kg, 475 ug/kg, 500 ug/kg, 525 ug/kg, 550 ug/kg, 575 ug/kg, 600 ug/kg, 625 ug/kg, 650 ug/kg, 675 ug/kg, 700 ug/kg, 725 ug/kg, 750 ug/kg, 775 ug/kg, 800 ug/kg, 825 ug/kg, 850 ug/kg, 875 ug/kg, 900 ug/kg, 925 ug/kg, 950 ug/kg, 975 ug/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 15 mg/kg, 20 mg/kg, 25 mg/kg, 30 mg/kg, 35 mg/kg, 40 mg/kg, 45 mg/kg, 50 mg/kg, 60 mg/kg, 70 mg/kg, 80 mg/kg, 90 mg/kg, or 100 mg/kg. In certain embodiments, GLYX-13 may be therapeutically effective with a range (e.g., an intravenous dose range) of about 1 to about 10 mg/kg, e.g., about 5 to about 10 mg/kg, e.g. about 1 mg/kg, about 5 mg/kg, or about 10mg/kg.

In some embodiments, any of the GLYX-13 dosages described herein can be administered on a less than daily basis, e.g., every other day (e.g., every two days); one or two times a week; one, two or three times a week; two or three times a week; twice weekly (e.g. every 3 days, every 4 days, every 5 days, every 6 days or e.g. administered with an interval of about 2 to about 3 days between doses); every three to four days; once a week; once every two weeks (bi-weekly); twice monthly; once a month, once every two months, once every thre months, once every four months, once every five months, once every six months, or even less often. In certain embodiments, GLYX-13 is administered at a frequency of once a week, twice a week, once every two weeks, or any combination thereof.

In certain embodiments GLYX-13 is administered at a range (e.g., an intravenous dose range) of about 1 to about 10 mg/kg, e.g., about 5 to about 10 mg/kg, e.g. about 1 mg/kg, about 5 mg/kg, or about 10mg/kg, and/or GLYX-13 is administered at a frcqucncy of once a week, once every two weeks, or any combination thereof.

The present disclosure contemplates "combination therapy," which includes (but is not limited to) co-administering an effective amount of GLYX-13 and one or more other biologically active agents (e.g., one or more other anti-depressant agents) as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Combination therapy is intended to embrace administration of multiple therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single tablet or capsule or i.v. solution having a fixed ratio of each therapeutic agent or in multiple, single tablets, capsules, or i.v. solutions for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent (e.g., GLYX -13) of the combination selected may be administered by intravenous injection while the other therapeutic agents of the combination may be administered orally. Alternatively, for example, all therapeutic agents may be administered orally or all therapeutic agents may be administered by intravenous injection.

GLYX-13 as well as any other pharmacological agent (e.g., one or more other antidepressant agents) of the present invention may be administered by various means, depending on their intended use, as is well known in the art. For example, if compositions of the present invention are to be administered orally, they may be formulated as tablets, capsules, granules, powders or syrups. Alternatively, formulations of the present invention may be administered parenterally as injections (intravenous, intramuscular or subcutaneous), drop infusion preparations, or suppositories. These formulations may be prepared by conventional means, and, if desired, the compositions may be mixed with any conventional additive, such as an excipient, a binder, a disintegrating agent, a lubricant, a corrigent, a solubilizing agent, a suspension aid, an emulsifying agent or a coating agent.

In some embodiments, GLYX-13 herein may be administered parenterally to a patient including, but not limited to, subcutaneously and intravenously. In some embodiments, one or more of the components of the combinations described herein may also be administered via slow controlled i.v. infusion or by release from an implant device.

In formulations of the subject invention, wetting agents, emulsifiers and lubricants, such as sodium lauryl sulfate and magnesium stearate, as well as coloring agents, release agents, coating agents, sweetening, flavoring and perfuming agents, preservatives and antioxidants may be present in the formulated agents.

Subject compositions may be suitable for oral, topical (including buccal and sublingual), rectal, vaginal, aerosol and/or parenteral administration. The formulations may conveniently be presented in unit dosage form and may be prepared by any methods well known in the art of pharmacy. The amount of composition that may be combined with a carrier material to produce a single dose vary depending upon the subject being treated, and the particular mode of administration.

Methods of preparing these formulations include the step of bringing into association compositions of the present invention with the carrier and, optionally, one or more accessory ingredients. In general, the formulations are prepared by uniformly and intimately bringing into association agents with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia), each containing a predetermined amount of a subject composition thereof as an active ingredient. Compositions of the present invention may also be administered as a bolus, electuary, or paste.

In solid dosage forms for oral administration (capsules, tablets, pills, dragees, powders, granules and the like), the subject composition is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the subject composition moistened with an inert liquid diluent. Tablets, and other solid dosage forms, such as dragees, capsules, pills and granules, may optionally be scored or prepared with coatings and shells, such as enteric coatings and other coatings well known in the pharmaceutical-formulating art.

Liquid dosage forms for oral administration include pharmaceutically acceptable emulsions, microemulsions, solutions, suspensions, syrups and elixirs. In addition to the subject composition, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylcnc glycol, oils (in particular, cottonseed, groundnut, com, germ, olive, castor and sesame oils), glycerol, tetrahydrofuryl alcohol, polyethylene glycols and fatty acid esters of sorbitan, cyclodextrins and mixtures thereof.

Suspensions, in addition to the subject composition, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions of this invention suitable for parenteral administration comprise a subject composition in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

"Pharmaceutically or pharmacologically acceptable" include molecular entities and compositions that do not produce an adverse, allergic or other untoward reaction when administered to an animal, or a human, as appropriate. For human administration, preparations should meet sterility, pyrogenicity, general safety and purity standards as required by FDA Office of Biologics standards. The term "pharmaceutically acceptable carrier" or "pharmaceutically acceptable excipient" as used herein refers to any and all solvents, dispersion media, coatings, isotonic and absorption delaying agents, and the like, that are compatible with pharmaceutical administration. The use of such media and agents for pharmaceutically active substances is well known in the art. The combinations described herein may also contain other active compounds providing supplemental, additional, or enhanced therapeutic functions. Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate and cyclodextrins. Proper fluidity may be maintained, for example, by the use of coating materials, such as lecithin, by the maintenance of the required particle size in the case of dispersions, and by the use of surfactants.

Disclosed compounds may be provided as part of a liquid or solid formulation, for example, aqueous or oily suspensions, solutions, emulsions, syrups, and/or elixirs. The compositions may also be formulated as a dry product for constitution with water or other suitable vehicle before use. Such liquid preparations may contain additives including, but not limited to, suspending agents, emulsifying agents, nonaqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl hydroxybenzoate and sorbic acid. Contemplated compounds may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

The present disclosure has multiple aspects, illustrated by the following non-limiting examples.

### EXAMPLES

### Example 1: Neural activation in hippocampal and related learning and memory regions

GLYX-13 was used to investigate whether this compound changed functional activation in hippocampal and related learning regions healthy young adults.

Methods Twenty-four healthy individuals underwent functional magnetic resonance imaging (fMRI) after randomization to IV-injection of 5 mg of GLYX-13 (n=12) or placebo (n=12). Groups were matched on demographic factors, estimated IQ, and dose volume. While undergoing scanning, subjects performed an item category association task in which they learned over repeated cycles which of two categories a series of numbers were assigned. Change in blood-oxygen level dependent (BOLD) activity was measured during correctly performed trials, and differences in activation between groups were compared across learning cycles.

Results Both groups demonstrated increased task accuracy over successive cycles indicating that they learned category membership. Compared to Placebo, the GLYX-13 group showed enhanced activation over learning cycles in several learning and memory regions including hippocampus, parahippocampal gyrus, and amygdala, as well as other regions including superior temporal, middle frontal, and inferior frontal gyri. There were no regions in which Placebo demonstrated greater activation compared to GLYX-13.

These findings suggest that GLYX-13 functionally influences neural regions involved in learning in humans, and thus may be a promising cognitive enhancer.

### Example 2: Study Showing that GLYX-13 Enhances Neural Activation in Learning and Memory Related Regions in Healthy Young Adults

We investigated whether a single IV administration of GLYX-13 compared to placebo changed functional activation among healthy young adults performing a learning and memory task while undergoing fMRI.

### Methods.

***Inclusion Criteria.*** The inclusion criteria for adult subjects participating in the study were as follows (i) 18-40 years of age; (ii) estimated IQ within what is considered to be the normal range (80-120); and (iii) no personal medical, neurologic, or psychiatric history, or reported history of psychiatric illness among first degree relatives.

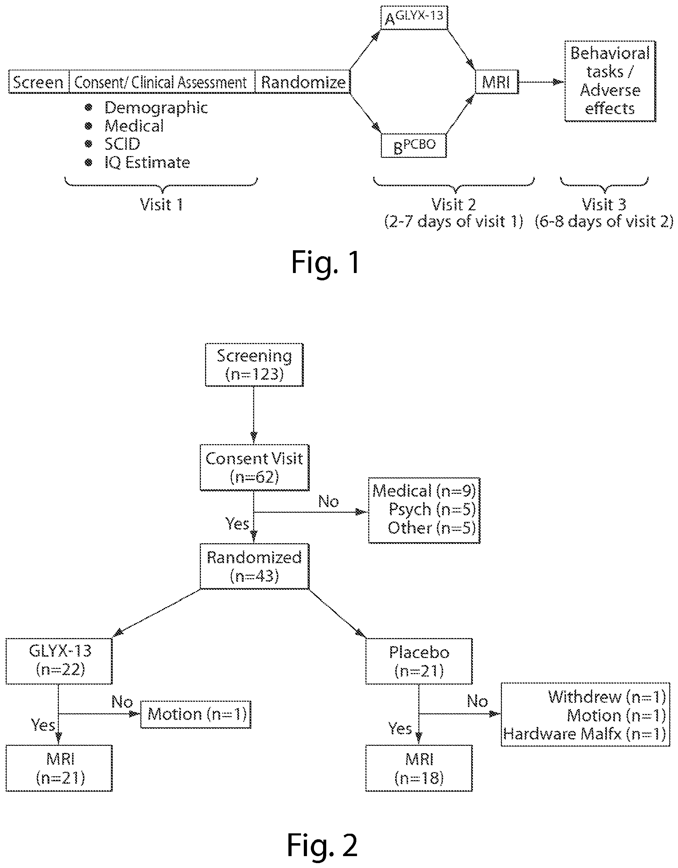

***Study Design*** (randomized, single-blind, parallel group). A flow chart showing the study design is shown in FIG. 1. After initial (Visit 1), patients were randomized to either single IV administration of GLYX-13 (5 mg/kg) or placebo on subsequent visit (Visit 2). fMRI studies started within 20 minutes post-infusion. Subjects returned approximately one week after Visit 2 to complete behavioral tasks in scanner and assess any adverse effects. The study flow is shown in FIG. 2, and the subject characteristics are summarized in Table 1.

**Table 1**

| **Subject characteristic** | **GLYX-13 (n=21)** | **Placebo (n=18)** | **p value** (Significance level from one way ANOVA for continuous measures or χ2 test for categorical measures) |
|---|---|---|---|
| Age (years) | 27.2 (4.5) | 25.2 (5.0) | 0.25 |
| Sex (M:F) | 10:11 | 9:9 | 0.52 |
| Race (Ca:AA:As) | 14:4:3 | 12:4:2 | 0.93 |
| Handedness | 19:2 | 16:2 | 0.72 |
| Estimated IQ | 105.7 (9.2) | 106.8 (8.8) | 0.71 |
| Dose Volume (mL) | 5.9 (0.8) | 5.9 (1.1) | 0.82 |
| Dose to fMRI acquisition time (min) | 82.4 (34/0) | 84.0 (33.8) | 0.88 |

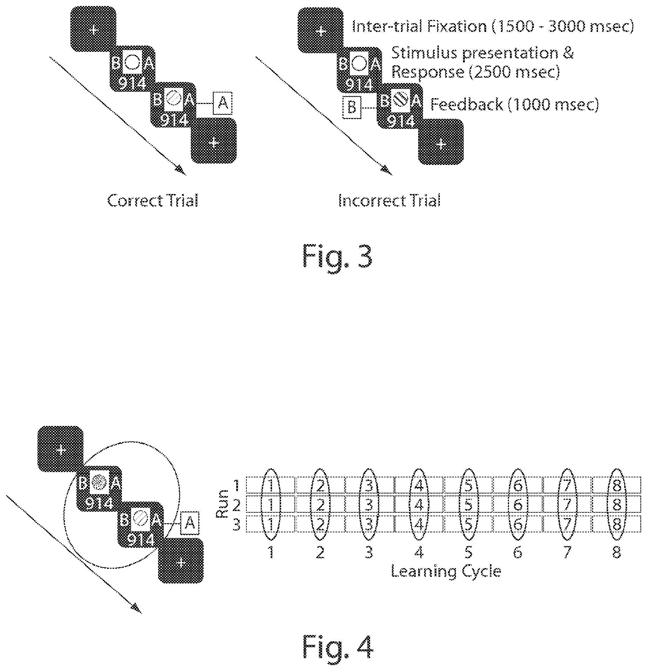

***Item Category Association Task*** (see, e.g., Onur OA, Schlaepfer TE, Kukolja J, Bauer A, Jeung H, Patin A, Otte D-M, Shah NJ, Maier W, Kendrick KM, Fink GR, Hurleman R (2010). The N-methyl-D-aspartate receptor co-agonist D-Cycloserine facilitates declarative learning and hippocampal activity in humans. Biol. Psychiatry, 67, 1205). Subjects were asked to learn arbitrary group membership (A or B) of 3-digit numbers. Visual feedback was provided immediately following choice (button press) to indicate correct item category association. There were eight category memberships to be learned, which were presented over eight cycles (i.e., 7 repititions) for a total of 64 trials per run; three sets of learning runs (each with different 3-digit number sets. See FIG. 3.

***Image Acquisition.*** 3T TIM Trio system (Siemens Medical Systems) with 32 channel coil. High resolution 3D T1-weighted MPRAGE sequence for spatial alignment and standardization (TE=3.16 msec, TR=2400 msec, 1 x 1 x 1 mm voxels; 8.09 min. acquisition time). fMRI (189 volumes collected consisting of 32 axial images acquired parallel to A-P commissure using an EPI sequence contrast per run (TE==20 msec, TR=2000msec, FOV=220 x 206mm, flip 80, 1.7 x 1.7 x 3.0 mm voxels); 6.40 min. acquisition time per functional run).

***Functional Analysis.*** Event related fMRI analysis conducted with FSL (FMRIB software library)'s FEAT tool (brain extraction tool (BET) used to remove non-brain tissue; high pass temporal filter applied with 100 msec cutoff; functional data corrected for head motion using MCFLIRT, transformed into MNI space and smoothed with Gaussian kernel of FWHM 5 mm; functional data registered to high-resolution structural scan and then transformed into standard MNI space). The first-level fixed effects analyses were performed on individual runs to model activation per learning cycle associated with corrcct, incorrect, and no response trials. The second-level fixed effects analyses combined individual subjects activation for learning cycle across three runs for correct trials. See FIG. 4. Mixed effect meta analysis (MEMA) in AFNI was used in whole brain analyses to model changes in BOLD activation during correctly performed trials as a function of learning cycle, thereby identifying circuitry supporting category learning on this task.

### Results

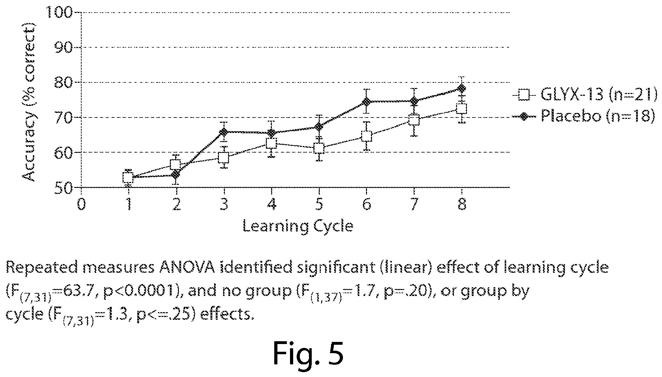

***Behavioral Task Performance.*** GLYX-13 and Placebo treated subjects show a robust and comparable learning effect across cycle. See FIG. 5.

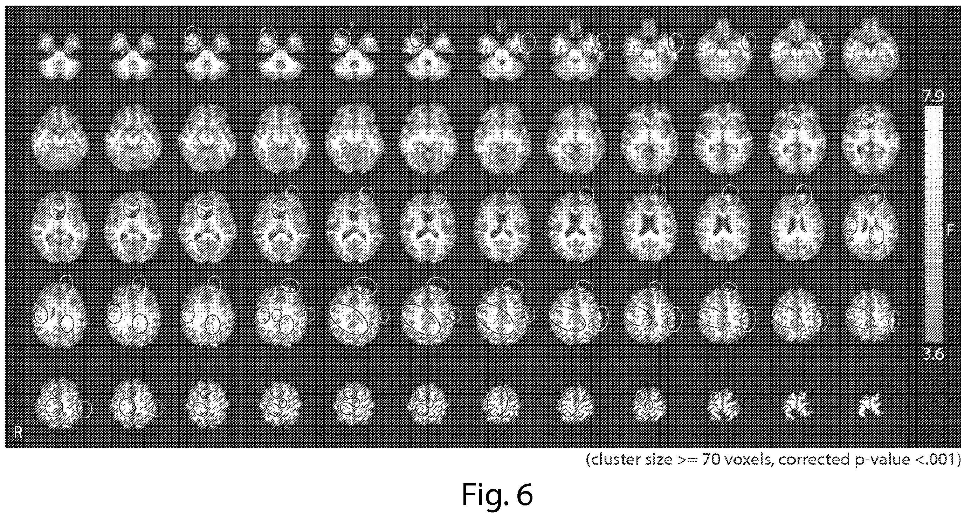

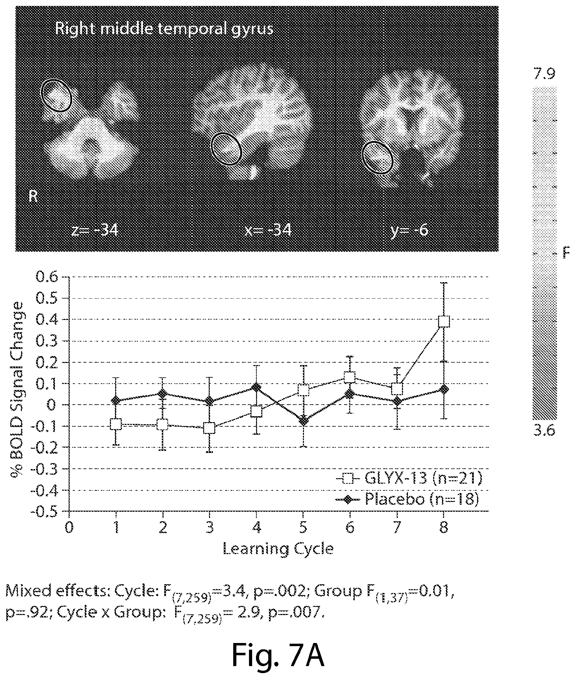

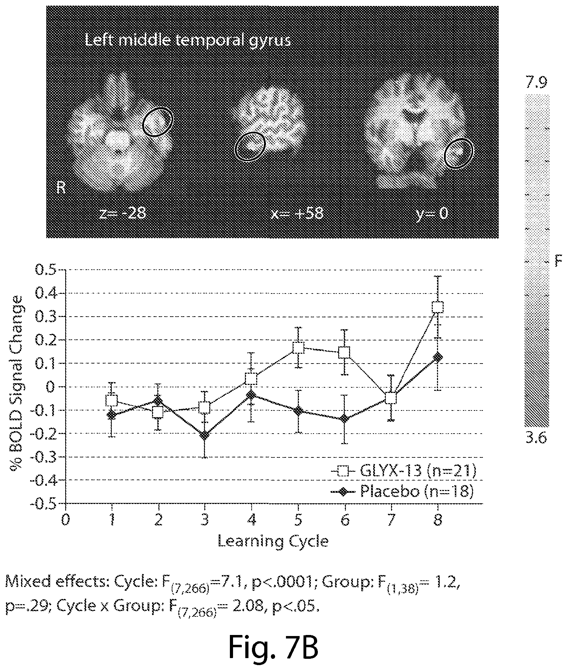

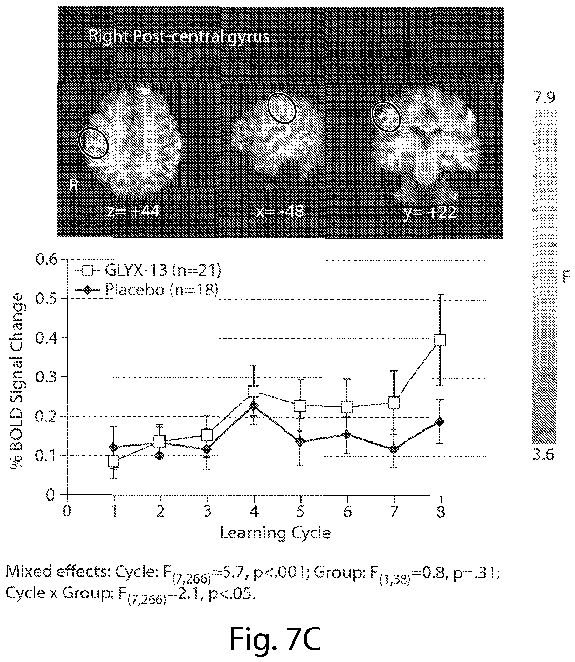

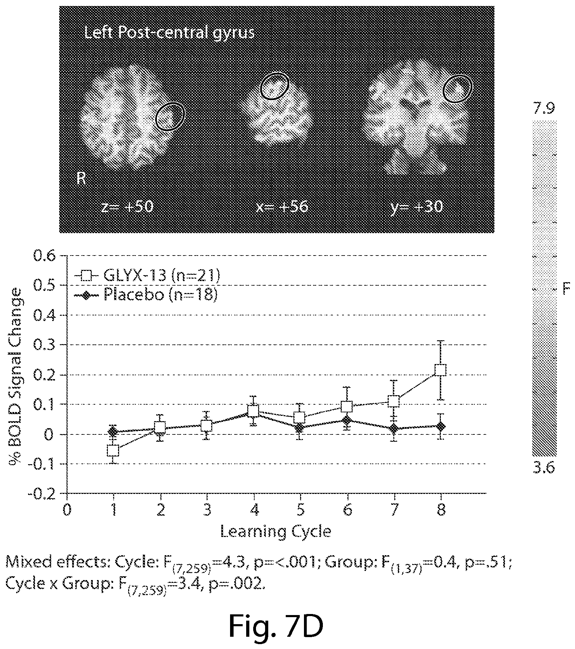

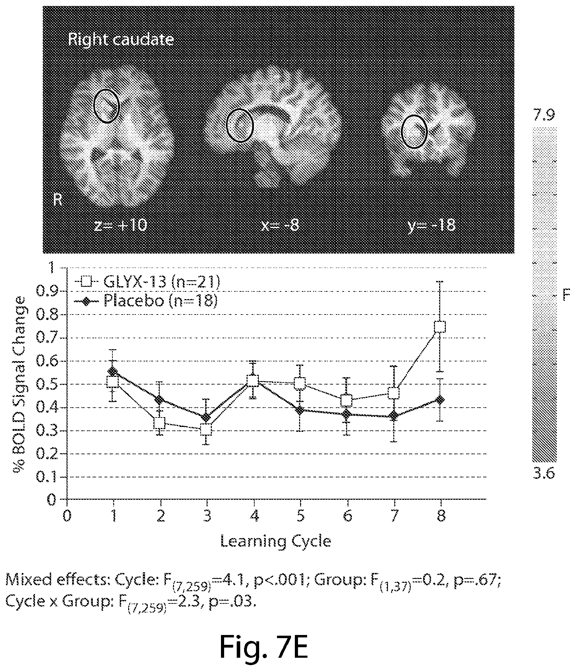

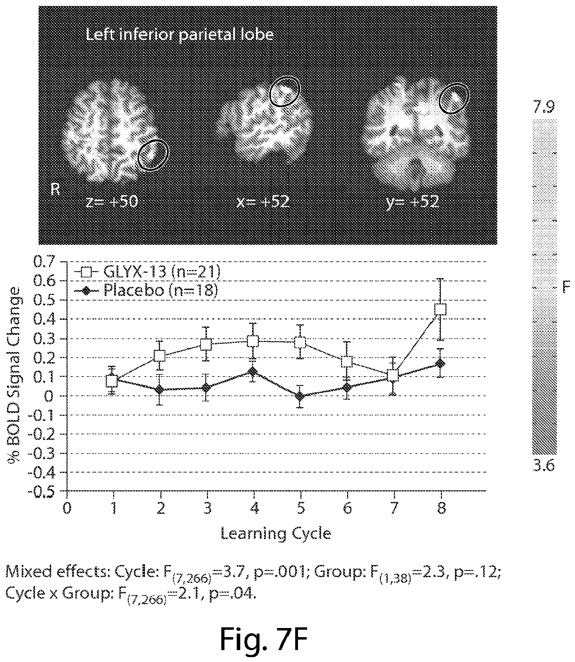

***fMRI Results.*** FIG 6 provides a series of fMRI images of regions showing significant change in BOLD activation across learning cycles. FIGS. 7A-7F provide a series of fMRI images of regions showing that among identified regions, six demonstrated significant group by cycle interaction effects on extracted mean % signal change, all of which demonstrated enhanced activation among GLYX-13 vs. Placebo treated individuals.

In the context of comparable performance, healthy individuals who received a single administration of GLYX-13 demonstrated an enhanced BOLD signal change in a task-elicited circuit relative to those individuals who received placebo. These results suggest that GLYX-13 functionally influences neural regions involved in learning and memory in healthy individuals.

### EQUIVALENTS

While specific embodiments of the subject disclosure have been discussed, the above specification is illustrative and not restrictive. Many variations of the disclosure will become apparent to those skilled in the art upon review of this specification. The full scope of the disclosure should be determined by reference to the claims, along with their full scope of equivalents, and the specification, along with such variations.

Unless otherwise indicated, all numbers expressing quantities of ingredients, reaction conditions, parameters, descriptive features and so forth used in the specification and claims are to be understood as being modified in all instances by the term "about." Accordingly, unless indicated to the contrary, the numerical parameters set forth in this specification and attached claims arc approximations that may vary depending upon the desired properties sought to be obtained by the present invention.

### INCORPORATION BY REFERENCE

All publications and patents mentioned herein, including those items listed below, are hereby incorporated by reference in their entirety as if each individual publication or patent was specifically and individually indicated to be incorporated by reference. In case of conflict, the present application, including any definitions herein, will control.

## Claims

1. A method of treating a cognitive disorder or enhancing cognitive function and/or learning in a patient in need thereof, comprising:
generating a MRI signal that is a measurement of brain activity in a patient;
identifying the signal as a normal or abnormal brain state of the patient; and
administering to the patient an effective amount of GLYX-13 based on the signal identification.

2. The method of claim 1, wherein the generating an MRI signal comprises using functional magnetic resonance imaging.

3. The method of claim 2, wherein the functional magnetic resonance imaging comprises blood-oxygen-level dependent contrast imaging.

4. The method of claim 1, wherein the brain activity is neural activation.

5. The method of claim 4, wherein the neural activation is neural activation in learning and/or memory related regions of the brain.

6. A method for identifying a biomarker related to neural activation, learning, or memory or identifying a patient population who is more susceptible to such disorders, comprising:
administering GLYX-13 to an animal;
imaging the animal using functional magnetic resonance to create measurable activity;
analyzing the activity; and
identifying the biomarker as result of the activity.

7. The method of claim 6, wherein using functional magnetic resonance to create measurable activity comprises using blood-oxygen-level dependent contrast imaging.

8. The method of claim 7, wherein the measurable activity comprises changes in blood flow and/or blood oxidation in one or more regions of the brain in the animal.

9. The method of claim 6 or 7, wherein the biomarker is a blood-oxygen-level dependent contrast signal in one or more regions of the brain in the animal.

10. The method of claim 6, wherein the method further comprises associating a patient or subpopulation of patients with the biomarker.

11. The method of claim 6, wherein the method further comprises one or both of the following: (a) determining whether GLYX-13 would be therapeutically effective for treating a cognitive or mental disorder; and (b) determining susceptibility/receptivity in patient or a subpopulation of patients suffering from a cognitive or mental disorder.

12. The method of claim 11, wherein the mental disorder is depression.

13. The method of claim 11, wherein the depression is refractory.

14. A method of treating a disorder selected from the group consisting of epilepsy, AIDS dementia, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, impulse control disorders, attention deficit disorder, attention deficit hyperactivity disorder, schizophrenia, anxiety, amelioration of opiate, nicotine and/or ethanol addiction, spinal cord injury, diabetic retinopathy, traumatic brain injury, post-traumatic stress syndrome, Huntington's chorea, Alzheimer's disease, memory loss that accompanies early stage Alzheimer's disease, depression conditions, Major Depressive Disorder, Dysthymic Disorder, Psychotic depression, Postpartum depression, Seasonal affective disorder (SAD), mood disorder, depressions caused by chronic medical conditions such as cancer or chronic pain, chemotherapy, chronic stress, Bipolar disorder, and manic depressive disorder, in a patient in need thereof, comprising:
generating a MRI signal that is a measurement of brain activity in a patient;
identifying the signal as a normal or abnormal brain state of the patient; and
administering to the patient an effective amount of GLYX-13 based on the signal identification.

15. The method of claim 14, wherein the generating an MRI signal comprises using functional magnetic resonance imaging.

16. The method of claim 15, wherein the functional magnetic resonance imaging comprises blood-oxygen-level dependent contrast imaging.

17. A method for identifying a biomarker related to a disorder selected from the group consisting of epilepsy, AIDS dementia, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, impulse control disorders, attention deficit disorder, attention deficit hyperactivity disorder, schizophrenia, anxiety, amelioration of opiate, nicotine and/or ethanol addiction, spinal cord injury, diabetic retinopathy, traumatic brain injury, post-traumatic stress syndrome, Huntington's chorea, Alzheimer's disease, memory loss that accompanies early stage Alzheimer's disease, depression conditions, Major Depressive Disorder, Dysthymic Disorder, Psychotic depression, Postpartum depression, Seasonal affective disorder (SAD), mood disorder, depressions caused by chronic medical conditions such as cancer or chronic pain, chemotherapy, chronic stress, Bipolar disorder, and manic depressive disorder, or identifying a patient population who is more susceptible to such disorders, comprising:
administering GLYX-13 to an animal;
imaging the animal using functional magnetic resonance to create measurable activity;
analyzing the activity, and
identifying the biomarker as result of the activity.

18. The method of claim 17, wherein using functional magnetic resonance to create measurable activity comprises using blood-oxygen-level dependent contrast imaging.

19. The method of claim 18, wherein the measurable activity comprises changes in blood flow and/or blood oxidation in one or more regions of the brain in the animal.

20. The method of claim 18 or 19, wherein the biomarker is a blood-oxygen-level dependent contrast signal in one or more regions of the brain in the animal.

21. The method of claim 17, wherein the method further comprises associating a patient or subpopulation of patients with the biomarker.

22. The method of claim 17, wherein the method further comprises one or both of the following: (a) determining whether GLYX-13 would be therapeutically effective for treating the disorder; and (b) determining susceptibility/receptivity in a patient or a subpopulation of patients suffering from the disorder.

23. A method for tracking treatment progress and/or treatment endpoints in a patient suffering from a cognitive or mental disorder comprising
generating a MRI signal that is a measurement of brain activity in a patient;
identifying the signal as a normal or abnormal brain state of the patient;
and administering to the patient an effective amount of GLYX-13 based on the signal identification.
